# EUROPEAN PATENT SPECIFICATION

(11) **EP 0 672 165 B1**
(45) Date of publication and mention of the grant of the patent: **07.01.1998**
(21) Application number: 93912796.5
(22) Date of filing: 26.05.1993
(51) Int. Cl.: C12P 41/00, C12P 7/64

(54) **A PROCESS FOR THE PREPARATION OF S-(+)-2-(3-BENZOYLPHENYL)PROPIONIC ACID BY ENZYME-CATALYSED ENANTIOSELECTIVE TRANSESTERIFICATION IN AN ORGANIC SOLVENT**
VERFAHREN ZUR HERSTELLUNG VON S-(+)-2-(-3BENZOYLPHENYL)PROPIONSÄURE DURCH ENZYM KATALYSIERTE ENANTHIOSELEKTIEVE UMESTERUNG IN EINEM ORGANISCHEN LÖSUNGSMITTEL.
PROCEDE DE PREPARATION D'ACIDE S-(+)-2-(3-BENZOYLPHENYLE)PROPIONIQUE PAR TRANSESTERIFICATION ENANTIOSELECTIVE CATALYSEE PAR VOIE ENZYMATIQUE DANS UN SOLVANT ORGANIQUE

(30) Priority: 08.06.1992 ES 9201190; 08.06.1992 ES 9201191
(43) Date of publication of application: 20.09.1995
(73) Proprietor: LABORATORIOS MENARINI S.A., 08912 Badalona (ES)
(72) Inventor: CARGANICO, Germano, E-08912 Badalona (ES); MAULEON CASELLAS, David, E-08912 Badalona (ES); PALOMER BENET, Albert, E-08912 Badalona (ES)
(74) Representative: Minoja, Fabrizio
(86) International application number: EP9301322
(87) International publication number: WO9325704

(56) References cited:
- EP-A- 64 855
- EP-A- 227 078
- EP-A- 461 043
- EP-A- 498 706

## Description

### FIELD OF THE INVENTION.

The present invention relates to a process for the preparation of optically active S-(+)-2-(3-benzoylphenyl)propionic acid (I) by resolution of the racemate via stereoselective transesterification in organic medium of a racemic 2-(3-benzoylphenyl)propionic acid reactive ester (II) with an achiral alcohol, catalysed by an extracellular lipase of microbial origin, followed by selective hydrolysis or extraction of the obtained esters.

### TECHNOLOGICAL BACKGROUND.

A number of antiinflammatory agents of the family of the aryl or alkylarylpropionic acids (i.e. naproxen, ibuprofen, flurbiprofen, etc.) are known, having a chiral center in the α-carbon and therefore existing in two optically different forms. The higher activity of the S enantiomer compared with that of the R one is also known [Muller N. et al., Fundam. Clin. Pharmacol. 4, 617 (1990)]. This is also the case of ketoprofen (S-(+)-2-(3-benzoylphenyl)propionic acid), the physiological activity of which mainly resides in the S enantiomer [Yamaguchi T. et al., Folia Pharmacol. Japon. 90, 295 (1987)].

The achievement of the chiral S-(+)-2-(3-benzoylphenyl)propionic acid (I) was approached both by enantioselective synthesis [Fadel A., Synlett. 1, 48 (1992), FR 2,659,968 (21.3.1991)] and by resolution of the racemate, and particularly remarkable is the resolution by diastereomeric crystallization of a (-)-1-phenylpropylamine salt. In this case, the product, which is obtained in a 22% yield after two recrystallizations, shows an optical purity higher than 99% (Nohira H. et al. EP 423,467].

Among the enzymatic methods for the optical resolution, only the stereoselective hydrolysis in aqueous medium has been applied to the preparation of S-(+)-2-(3-benzoylphenyl)propionic acid. The contribution by Sih et al. [Sih C.L. et al., EP 227,078] should be outlined, in which the resolution of this compound by hydrolysis of the methyl ester thereof, catalysed by a purified form of the lipase from Candida cylindracea, with optical purity values higher than 95% and in a 78% yield, is described. In spite of these results, the procedure does not seem to be suitable for the preparative scale, due to the need to modify-purify the enzyme. On the other hand, Cerbelaud D. et al. [EP 330529 and FR 2 626 288] describe the resolution by enantioselective hydrolysis of the nitrile and the corresponding amide catalysed by microorganisms such as Corynebacterium I-774 or Brevibacterium R-312, to obtain the acid in an 88% yield and with an optical purity higher than 95%, even though it should be noted the disadvantage that these reactions are carried out at a high dilution (0.05 M). In both cases, the optically pure acid is obtained extracting with organic solvents the crude product from the hydrolysis, after alkalinization, to leave the ester in the organic phase and the acid in the aqueous basic one.

The enantioselective esterification and transesterification reactions catalysed by lipases have been applied to halo or aryloxy propionic acids substituted at the α-carbon.

The achievement of chiral 2-chloro or 2-bromopropionic acids by selective esterification catalysed by lipase from Candida cylindracea has been described to give better results in organic medium, i.e. in n-hexane [Klibanov A.M. et al., US 4,601,987; Kirchner G. et al., J. Am. Chem. Soc., 107 (24) 7072 (1985)], than in a water-organic solvent biphasic medium [Cambou et al. Biotech. & Bioeng., 26, 1449 (1984)]. Alternatively, these chiral acids are obtained by stereoselective transesterification catalysed by lipases from Penicillium cyclopium or Geotricum candidum. In practice, the α-chloropropionic acid ethyl ester is enantioselectively converted into the butyl ester in the presence of butanol, in a 10% conversion in 48 hours and a 68% enantiomeric excess of the resulting product [Aviron-Violet P, EP 396447]. Similarly, the trichloroethyl ester transesterifies in the presence of hexadecanol in a 50% conversion in 50 hours to obtain the chiral hexadecyl ester [Klibanov A.M. et al., US 4601987; Kirchner G. et al., J. Am. Chem. Soc. 107, 7072 (1985)]. In both cases, the separation of the final esters is carried out by distillation. Wu et al. described the resolution of 2-(4-chlorophenoxy)propionic acid via enantioselective esterification with n-butanol, catalysed by the lipase from Candida cylindracea (Meito-Sangyo Ltd. Japan ; 72 hours, 38% conversion, 86% optical purity of the n-butyl ester) in an n-hexane solution containing 2% (v/v) of phosphate buffer (0.2 M, pH 7.0) [Wu S.-H., Chu F.-Ed., Wang K.- T. Bioorganic & Med. Chem. Letters 1 (7) 339 (1991)]

EP-A-0461043 discloses the use of water soluble esters of arylpropionic acids as substrates for transesterification, this latter being carried out in water or in a water/alcohol mixture, differently from the present patent application, which reports the use of water immiscible organic solvent.

### DISCLOSURE OF THE INVENTION

The present invention relates to a process for the preparation of chiral optically pure S-(+)-2-(3-benzoylphenyl)propionic acid (I) 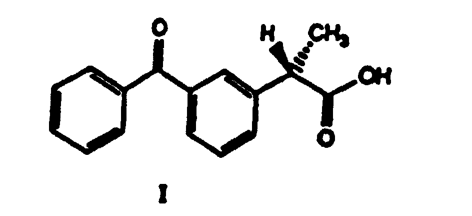 by resolution of the racemate of a reactive ester (II) 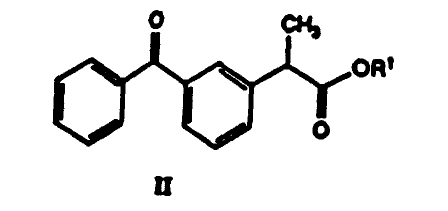 of said compound via enantioselective transesterification in organic medium, catalysed by an extracellular lipase of microbial origin.

In formula (II), R¹ is a straight, branched or cyclic C₁-C₁₀ alkyl group, substituted by one or more electron-withdrawing groups, for example halogen, nitro, cyano, -OR², -SR², -COR² [wherein R² can be C₁-C₆ alkyl or cycloalkyl], NR³R⁴ [wherein R³ and R⁴ can be H, C₁-C₆ straight, branched or cyclic alkyl, or they can form a C₄-C₆ cycle together with the nitrogen and optionally other heteroatoms]. The electron-withdrawing group can be in the α- or β- positions of the R¹ group, thus the ester being referred to as "reactive" ester due to the faster hydrolysis rate observed for said derivatives. Some specific examples of R¹ groups are 2-chloroethyl, 2,2,2,-trichloroethyl, 2-fluoroethyl, 2,2,2-trifluoroethyl, cyanomethyl, 2-nitropropyl, 2-aminoethyl, N,N-dimethyl-2-aminoethyl.

The process consists in subjecting the substrate, the reactive ester (II), to the action of an extracellular carboxylesterase (also named "lipase", EC 3.1.1.3.) of microbial origin, as described in the following scheme.

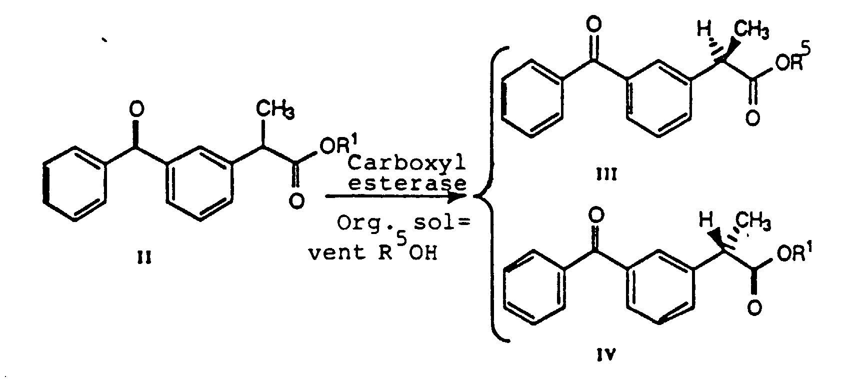

The reaction is carried out in a water-immiscible organic solvent such as a straight or branched aliphatic ether, or an aliphatic or aromatic hydrocarbon, which can be halogenated or not, and the like, in the presence of the transesterification alcohol, which has a R⁵OH structure, wherein R⁵ is a C₁-C₈ straight, branched or cyclic alkyl group, for example, n-butanol or n-octanol, or R⁵ is a C₁-C₄ straight, branched or cyclic alkyl group substituted with a group having a second basic or acid functionality, for example, a nitro-genated heteroaryl group [such as pyridyl, pyrazolyl, imidazolyl, thiazolyl, etc] or a -NR⁶R⁷ group [wherein R⁶ and R⁷ can be H, C₁-C₄ straight, branched or cyclic alkyl, or they can form a C₄-C₆ cycle together with the nitrogen and optionally other heteroatoms], a -CO₂R⁸, -SO₃R⁸, -PO₃R⁸R⁸ group [wherein R⁸ can be H or an alkali metal]. Some specific examples of these R⁵OH difunctional alcohols are 2-(2-pyridyl)ethanol, N,N-diethylethanol and 2-hydroxyethylsulfonic, glycolic and lactic acids.

Among the carboxylesterases (also named "lipases") capable to catalyse this type of reactions, the derivatives from microorganisms of the genus Candida, Rhizopus, Mucor, Aspergillus, Penicillium and Pseudomonas showed a special activity, particularly the lipase from Mucor meihei commercialized by Amano Pharm. Co. Ltd. (Nagoya, JP) under the commercial name Amano M-AP-10 and the lipase isolated from the same microorganism by NOVO Nordisk Ind. (Bagsvaerdt, DK) under the name Lipozyme 10000. In all cases, a preference for the R enantiomer of the ester was observed, as the alkyl ester (III) of R configuration is obtained, whereas the enantiomer S thereof remains in form of the starting reactive ester (IV).

The concentration of the starting racemic ester (II) can be from 0.05 to 1.0 M, preferably 0.1-0.3 M. The molar ratio of ester to transesterification alcohol is at least 1:0.5; the latter may be used as solvent. The working temperature is from 0 to 60°C, but preferably the reaction is carried out at 20-30°C. The reaction time is from 2 to 15 days, preferably 7 days. In some cases, the reaction will be carried out until conversions higher than 50% (approx. 55%), since, according to the general theory of the irreversible enzyme reactions, [C.S. Chen, Angew. Chem. Int. Ed. Engl., 28, 695 (1989)], and since the interesting compound (S isomer) is the remaining substrate, the optical purity thereof can be optimized only excessing a 50% conversion.

The separation of the desired ester from the crude transesterification product when R⁵ is a C₁-C₈ alkyl group is conveniently carried out by subjecting the mixture to chemoselective hydrolysis with aqueous alkali, at such a pH that only the reactive ester IV is hydrolysed; this pH can be from 7 to 12, preferably from 9 to 10, kept constant by means of a pH-Stat, or in the suitable buffer solution. Moreover, the reaction mixture can be added with a water-miscible co-solvent in order to increase the solubility of these compounds in the medium. Preferably, co-solvents such as tetrahydrofuran, dioxane, aliphatic alcohols, dimethylformamide or acetone will be used.

Once the reaction mixture is treated, it is alkalinized and the resulting acid (I) is separated from ester (III) by extraction with a water-immiscible organic solvent, such as ethyl acetate, diethyl ether, toluene, and the like, or by centrifugation or filtration of the resulting suspension to obtain the R-ester (III), whereas the acidification gives the optically active S-(+)-2-(3-benzoylphenyl)propionic acid (I).

Alternatively, when R⁵ is a C₁-C₄ alkyl substituted by basic or acid groups, the separation of both esters (III) and (IV) in the crude transesterification product is carried out by partition between a water-immiscible organic solvent (such as ethyl acetate, diethyl ether, toluene, etc) and an aqueous solution at the pH suitable to dissolve the ester (III), leaving the starting ester (IV) in the organic phase. The pH optimum will depend on the nature of the bifunctional alcohol used, for example, in case (III) contains a pyridyl group in R⁵, this pH ranges from 0 to 4, whereas if R⁵ contains a carboxylic or sulfonic acid, it is from 6 to 14, preferably 9 to 14, so that the ester (III) is completely dissolved in water.

The remaining reactive ester (IV), once separated, is subjected to chemical or enzymatic hydrolysis: The chemical hydrolysis is carried out at alkaline pH which can range from 7 to 12, preferably from 9 to 10, pH being kept constant either by a pH-Stat or by a suitable buffer solution. Moreover, the reaction mixture can be added with a water-miscible co-solvent in order to increase the solubility of the ester (IV) in the medium and therefore the hydrolysis reaction rate. Preferably, co-solvents such as tetrahydrofuran, 1,4-dioxane, aliphatic alcohols, N,N-dimethylformamide and the like will be used. Alternatively, the hydrolysis may be carried out using a lipase, esterase or any other hydrolase at the optimum pH thereof. Finally, the resulting acid (I) is separated by acidification and extraction with a water-immiscible organic solvent such as those indicated above, or by centrifugation or filtration of the resulting suspension to obtain a solid which is S-(+)-2-(3-benzoylphenyl)propionic acid (I).

The following examples further illustrate the invention.

### Example 1a: Transesterification reaction of the ester (II) (R¹ = -CH₂CF₃) with n-butanol catalysed by lipase from Mucor meihei, Amano MAP-10

In a 5000 ml Erlenmeyer flask fitted with mechanical stirrer, 150 g (0.446 mole) of racemic trifluoroethyl ester (I) (R¹ = -CH₂CF₃) and 66.1 g (0.892 mole) of n-butanol are dissolved in 3000 ml of dry diisopropyl ether and 6.0 g of 4Å molecular sieves are added. Subsequently 223 g of lipase from Mucor meihei (Amano MAP-10) are added, to obtain a suspension which is stirred for 7 days at 27°C. The final mixture is filtered, the filtrate is washed with 0.1 N NaOH, dried with magnesium sulfate and the solvent is evaporated off under vacuum to obtain 189 g of a crude product which is analyzed by HPLC, showing a 47.3% content of trifluoroethyl ester (IV) (R¹ = -CH₂CF₃) of a 91.8% optical purity, together with 48.7% of n-butyl ester (III) (R¹ = -O(CH₂)₃CH₃). The crude reaction product is used in the subsequent step without further purification.

The optical purity of the chiral compounds in this and in the following examples was determined by high performance liquid chromatography (HPLC) by means of a Chiralcel OJ column manufactured by Daicel Chem. Ind., Ltd. and a n-hexane/i-propanol/AcOH (80/19.5/0.5) mobile phase.

### Example 1b: Chemoselective hydrolysis of the trifluoroethyl ester (IV) (R¹ = -CH₂CF₃)

50 g of the crude product of the above reaction (la) are dissolved in 4800 ml of water/1,4-dioxane (80:20 v/v). The mixture is adjusted to pH 9.5, which pH is kept for 6 days by addition of 0.1 N NaOH in a pH-Stat. The volume is reduced to 1/5 by evaporation under vacuum and pH is adjusted to 12 by addition of 1 N NaOH. The solid is dissolved in diethyl ether, washed with a NaCl saturated solution, dried and the solvent is evaporated off under vacuum. 25 g of the n-butyl ester (III) (R⁵ = -O(CH₂)₃CH₃) are obtained with a 94% optical purity. The aqueous filtrate is brought to pH 1 by addition of 1 N HCl, extracted with ethyl ether (5 x 200 ml) and dried over magnesium sulfate. After evaporation of the solvent under vacuum, 13.6 g (91% yield) of S-(+)-2-(3-benzoylphenyl)propionic acid (I) are obtained of 87% optical purity. 1H N.M.R. (CDCl₃, 300 MHz) 7.35-7.80 (m, 9H; pH-H); 3.77 (q J=4, lH; CHCOOH); 1.50 (d J=4, 3H; CH₃); IR (CHCl₃) 3000-3400, 1730, 1650, 1600, 1460, 1410, 1290, 1200, 1150, 1075 cm-1; [α]²³_{D} = +49.70° (c 0.93, CHCl₃). n-Butyl ester (III) (R⁵ = (CH₂)₃CH₃): 1H N.M.R. (CDCl₃, 300 MHz) 7.4-7.9 (m, 9H; Ph-H); 4.25 (m, 2H; COOCH2CH2); 3.88 (dd J=3.7, lH; CHCOO); 1.56 (d J=3, 3H; CHCH3), 1.53 (m, 3H; CH₂CH₃).

### Example 2: Transesterification reaction of the trifluoroethyl ester (I) (R¹ = -CH₂CF₃) with n-butanol catalysed by lipase from Mucor meihei, Lipozyme 10000

In a 100 ml Erlenmeyer flask fitted with magnetic stirrer, 2.0 g (5.95 mmoles) of trifluoroethyl ester (I) (R¹ = -CH₂CF₃) and 441 mg (5.45 mmoles) of n-butanol are dissolved in 60 ml of n-hexane. Subsequently, 1.19 g of lipase from Mucor meihei (Lipozyme 10000) are added to obtain a suspension which is stirred for 9 days at 27°C. The final mixture is centrifuged, washed with 0.1 N Na₂CO₃, dried with magnesium sulfate and the solvent is evaporated off under vacuum to obtain 1.71 g of a crude product which is analyzed by chiral HPLC, showing to contain 46.0% trifluoroethyl ester (IV) (R¹ = -CH₂CF₃) of a 90.3% optical purity, together with 49.7% of n-butyl ester (III) (R⁵ = -O(CH₂)₃CH₃) corresponding to a 51.9% conversion.

### Example 3a) Transesterification of the ester (II) with 2-(2-pyridyl)ethanol catalysed by lipase of Mucor meihei.

In a 2 1 flask, 75 g (0.22 mole) of ester (II) (R¹ = -CH₂CF₃) and 22 g (0.18 mole) of 2-(2-pyridyl)ethanol are dissolved in 885 ml of diisopropyl ether. Subsequently 112 g of lipase of Mucor meihei (Amano MAP-10^{R}) are added, to obtain a suspension which is stirred for 11 days at 27°C. The final mixture is filtered and the filtrate is extracted with 0.1 N HCl (5 x 200 ml). The organic phase is washed with 0.1 N NaOH (3 x 50 ml), dried over magnesium sulfate and the solvent is evaporated off under vacuum to obtain 40.8 g of the trifluoroethyl ester (IV) (R¹ = -CH₂CF₃), with a 93.5 % optical purity . The aqueous phase is adjusted to alkaline pH with 0.1 N NaOH and extracted with diethyl ether (5 x 100 ml), dried over magnesium sulfate and the solvent is evaporated off under vacuum to obtain 28.9 g of the 2-(2-pyridyl)ethyl ester (III) (R³ = -(CH₂)₂Py) with an optical purity higher than 99 %, which means a 41% conversion. 2-(2-Pyridyl)ethyl ester (III) (R³ = -(CH₂)₂Py): optical purity >99 %; 1H N.M.R. (300 MHz, CDCl₃): 8.45 (d J=3, lH; Py-H), 6.9-7.8 (m, 12H; Ar-H), 4.45 (m, 2H; COOCH), 3.7 (dd J=7.3, 2H; CH₃CH), 3.0 (dd J=7.3, 2H; CH₂Py), 1.45 (d J=3, 3H; CH₃). Trifluoroethyl ester (IV) (R¹ = CH₂CF₃): optical purity = 93.5 %; 1H N.M.R. (300 MHz, CDCl₃): 7.4-7.9 (m, 9H; Ph-H); 4.35 (m, 2H; CH₂CF₃); 3.88 (dd J=3.7, lH; CHCOO); 1.56 (d J=3, 3H; CH₃).

### Example 3b: Hydrolysis of the trifluoroethyl ester (IV) (R¹ = -CH₂CF₃).

1.5 g of trifluoroethyl ester (IV) of example 3a (93.5 % optical purity) are dissolved in 80 ml of a THF/water (50 % v/v) mixture. The mixture is brought to pH 9.5 and it is kept at this pH for 6 days, by addition of 0.1 N NaOH in a pH-Stat. The volume of the suspension is reduced to 1/2 by evaporation under vacuum and the suspension is acidified to pH = 1 by addition of 0.1 N HCl, extracted with diethyl ether (5 x 5 ml), dried and the solvent is evaporated off under vacuum. 1.0 g of S-(+)-2-(3-benzoylphenyl)propionic acid (90 % yield) is obtained, with a 91 % optical purity. 1H N.M..R. (CDCl₃, 300 MHz) 7.35-7.80 (m 9H, Ph-H); 3.77 (md J=4, lH; CHCH₃); 1.50 (d J=4, 3H; CHCH₃); IR (CHCl₃) 3000-3400, 1730, 1650, 1600, 1460, 1410, 1290, 1200, 1150, 1075 cm-1; M.p. =87-92°C; [α]D = +51.96° (c 1.0, CHCl₃).

## Claims

1. A process for the preparation of S-(+)-2-(3-benzoylphenyl)propionic acid, according to formula (I), 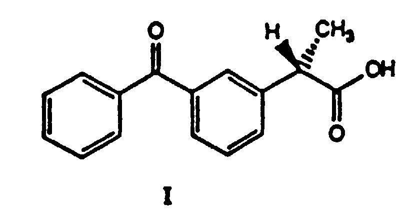 which process comprises the following steps:
a) stereoselective transesterification in organic medium, catalysed by a carboxylesterase enzyme of microbial origin, of an ester of formula (II) 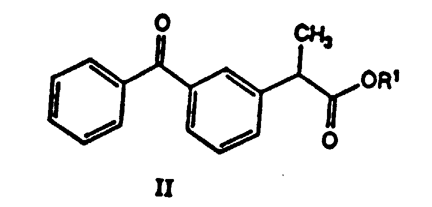 in which R¹ is a C₁-C₁₀ straight, branched or cyclic alkyl group, which can be substituted at the α- or β-positions by an electron-withdrawing group, said transesterification reaction is carried out in a water-immiscible organic solvent such as a straight or branched aliphatic ether or an aliphatic or aromatic hydrocarbon, which can be halogenated or not, with an alcohol of general formula R⁵OH, R⁵ being a C₁-C₈ straight, branched or cyclic alkyl group, or a C₁-C₄ straight, branched or cyclic alkyl group substituted by a group having acid or basic functionality; b) selective hydrolysis or extraction of ester of formula IV 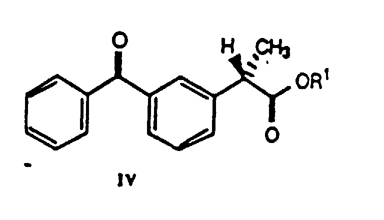 [wherein R¹ has the same meaning as in claim 1a] which is then converted to I.

2. A process according to claim 1, wherein the concentration of the starting ester (II) is from 0.05 to 1.0 M, the molar ratio of ester (II) to alcohol R⁵OH is at least 1:0.5; the reaction being carried out at a temperature from 0 to 60°C, for a time from 2 to 15 days.

3. A process according to claim 1 or 2, in which the electron-withdrawing group present in R¹ is halogen, nitro, cyano, -OR², -SR², -COR² [wherein R² can be C₁-C₆ alkyl or cycloalkyl], NR³R⁴ [wherein R³ and R⁴ can be H, C₁-C₆ straight, branched or cyclic alkyl, or they can form a C₄-C₆ cycle together with the nitrogen and optionally other heteroatoms].

4. A process according to any one of the above claims, in which R¹ is -CH₂CF₃, -CH₂CH₂F, -CH₂CH₂Cl or -CH₂CCl₃.

5. A process according to any one of the above claims, in which R¹ is -CH₂CF₃.

6. A process according to any one of the above claims, in which the enzyme used is the lipase from Mucor meihei.

7. A process according to any one of the above claims in which the R⁵OH alcohol is a C₁-C₈ linear, branched or cyclic alkanol and the ester IV [wherein R¹ has the same meaninig as in claim la] is chemoselectively hydrolysed by treatment with aqueous alkali at constant pH from 7 to 12.

8. A process according to claim 7, in which the alcohol of general formula R⁵OH is n-butanol.

9. A process according to claim 7 or 8, in which the resulting final S-acid and the R-ester are separated by extraction with a water-immiscible organic solvent or by centrifugation or filtration of the resulting suspension.

10. A process according to any one of the claims 1 to 6 above in which R⁵OH is a C₁-C₄ alkanol substituted by groups having acid or basic functionality and the unreacted ester IV [wherein R¹ has the same meaning as in claim la] having S-configuration is separated by partition between a water-immiscible organic solvent and an aqueous medium at a pH suitable to dissolve the formed ester of formula III 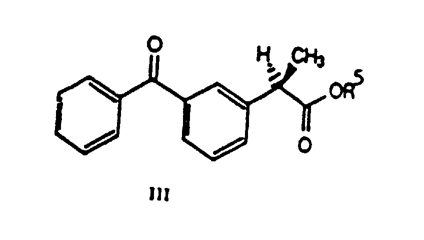 or by centrifugation or filtration of the resulting suspension followed by chemical or enzymatic hydrolysis of the ester IV.

11. A process according to claim 10 in which the R⁵OH alcohol is 2-(2-pyridyl)ethanol.

12. A process according to claim 10 or 11, in which the resulting esters are separated by partition between a water-immiscible organic solvent and an aqueous solution at acid pH from 0 to 4 or basic pH from 9 to 14.

## Patentansprüche

1. Verfahren zur Herstellung von S-(+)-2-(3-Benzoylphenyl)propionsäure der Formel (I) 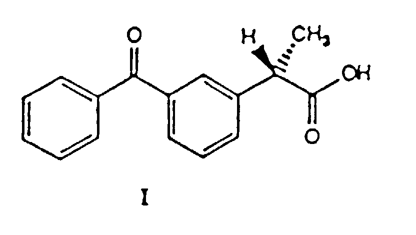 umfassend die folgenden Stufen:
a) stereoselektive Umesterung in einem organischen Medium eines Esters der Formel (II) 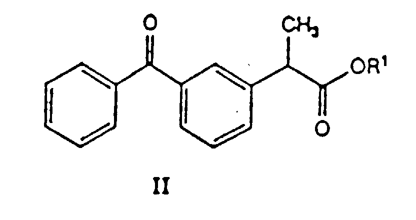 worin R¹ für eine geradkettige, verzweigte oder cyclische C₁-C₁₀-Alkylgruppe steht, die in α- oder β-Position durch eine Elektronen-entziehende Gruppe substituiert sein kann, die durch ein Carboxylesteraseenzym mikrobieller Herkunft katalysiert wird, wobei die Umesterungsreaktion in einem mit Wasser nicht mischbaren organischen Lösungsmittel, wie einem geradkettigen oder verzweigten aliphatischen Ether oder einem aliphatischen oder aromatischen Kohlenwasserstoff, der halogeniert sein kann oder nicht, mit einem Alkohol der allgemeinen Formel R⁵OH durchgeführt wird, wobei R⁵ für eine geradkettige, verzweigte oder cyclische C₁-C₈-Alkylgruppe oder eine geradkettige, verzweigte oder cyclische C₁-C₄-Alkylgruppe, die durch eine Gruppe mit saurer oder basischer Funktionalität substituiert ist, steht;
b) selektive Hydrolyse oder Extraktion eines Esters der Formel (IV) 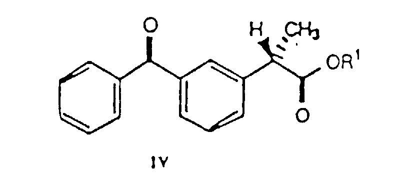 [worin R¹ die gleiche Bedeutung wie in Anspruch 1a) hat], der sodann zu I umgewandelt wird.

2. Verfahren nach Anspruch 1, dadurch **gekennzeichnet,** daß die Konzentration des Ausgangsesters (II) 0,05 bis 1,0 M beträgt, daß das Molverhältnis von Ester (II) zu Alkohol R⁵OH mindestens 1:0,5 beträgt, daß die Reaktion bei einer Temperatur von 0 bis 60°C über einen Zeitraum von 2 bis 15 Tagen durchgeführt wird.

3. Verfahren nach Anspruch 1 oder 2, dadurch **gekennzeichnet,** daß die in R¹ vorhandene Elektronen-entziehende Gruppe Halogen, Nitro, Cyano, -OR², -SR², -COR² [wobei R² für C₁-C₆-Alkyl oder Cycloalkyl stehen kann], NR³R⁴ [wobei R³ und R⁴ für H, geradkettiges, verzweigtes oder cyclisches C₁-C₆-Alkyl stehen können oder diese einen Teil eines C₄-C₆-Zyklus zusammen mit dem Stickstoff- und gegebenenfalls weiteren Heteroatomen sein können] ist.

4. Verfahren nach einem der vorstehenden Ansprüche, dadurch **gekennzeichnet,** daß R¹ -CH₂CF₃, -CH₂CH₂F, -CH₂CH₂Cl oder -CH₂CCl₃ ist.

5. Verfahren nach einem der vorstehenden Ansprüche, dadurch **gekennzeichnet,** daß R¹ -CH₂CF₃ ist.

6. Verfahren nach einem der vorstehenden Ansprüche, dadurch **gekennzeichnet,** daß das verwendete Enzym Lipase von Mucor meihei ist.

7. Verfahren nach einem der obigen Ansprüche, dadurch **gekennzeichnet**, daß der Alkohol R⁵OH ein lineares, verzweigtes oder cyclisches C₁-C₈-Alkanol ist und daß der Ester IV [worin R¹ die gleiche Bedeutung wie in Anspruch la) hat] chemoselektiv durch Behandlung mit wäßrigem Alkali bei einem konstanten pH-Wert von 7 bis 12 hydrolysiert wird.

8. Verfahren nach Anspruch 7, dadurch **gekennzeichnet,** daß der Alkohol der allgemeinen Formel R⁵OH n-Butanol ist.

9. Verfahren nach Anspruch 7 oder 8, dadurch **gekennzeichnet,** daß die am Ende resultierende S-Säure und der R-Ester durch Extraktion mit einem mit Wasser nicht mischbaren organischen Lösungsmittel oder durch Zentrifugieren oder Filtrieren der resultierenden Suspension getrennt werden.

10. Verfahren nach einem der vorstehenden Ansprüche 1 bis 6, dadurch **gekennzeichnet,** daß R⁵OH ein C₁-C₄-Alkanol ist, das durch Gruppen mit saurer oder basischer Funktionalität substituiert ist, und daß der nichtumgesetzte Ester IV [worin R¹ die gleiche Bedeutung wie in Anspruch 1a) hat] mit S-Konfiguration durch Aufteilung zwischen einem mit Wasser nicht mischbaren organischen Lösungsmittel und einem wäßrigen Medium bei einem geeigneten pH-Wert, um den gebildeten Ester der Formel III 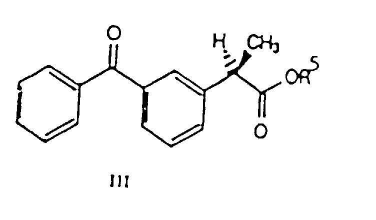 aufzulösen oder durch Zentrifugieren oder Filtrieren der resultierenden Suspension, gefolgt von chemischer oder enzymatischer Hydrolyse des Esters IV, abgetrennt wird.

11. Verfahren nach Anspruch 10, dadurch **gekennzeichnet,** daß der Alkohol R⁵OH 2-(2-Pyridyl)ethanol ist.

12. Verfahren nach Anspruch 10 oder 11, dadurch **gekennzeichnet,** daß die resultierenden Ester durch Aufteilung zwischen einem mit Wasser nicht mischbaren organischen Lösungsmittel und einer wäßrigen Lösung bei einem sauren pH-Wert von 0 bis 4 oder einem basischen pH-Wert von 9 bis 14 getrennt werden.

## Revendications

1. Un procédé pour la préparation de l'acide S-(+)-2-(3-benzoylphényl)propionique répondant à la formule (I), 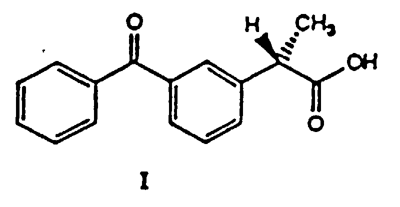 lequel procédé comprend les étapes suivantes :
a) trans-estérification stéréosélective dans un milieu organique, catalysée par une enzyme carboxylestérase d'origine microbienne, d'un ester de formule (II) 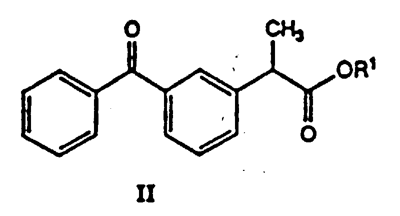 où R¹ est un groupe alkyle linéaire, ramifié ou cyclique en C₁-C₁₀, qui peut être substitué aux positions α ou β par un groupe attirant les électrons, ladite réaction de trans-estérification étant effectuée dans un solvant organique non miscible à l'eau tel qu'un éther aliphatique linéaire ou ramifié ou un hydrocarbure aliphatique ou aromatique, qui peut être halogéné ou non, avec un alcool de formule générale R⁵OH, R⁵ étant un groupe alkyle linéaire, ramifié ou cyclique en C₁-C₈ ou un groupe alkyle linéaire, ramifié ou cyclique en C₁-C₄ substitué par un groupe ayant une fonctionnalité acide ou basique ;
b) hydrolyse ou extraction sélective d'un ester de formule IV 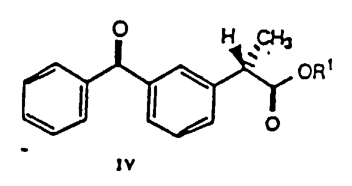 (où R¹ a la même signification que dans la revendication 1a), qui est ensuite converti en I.

2. Un procédé selon la revendication 1, dans lequel la concentration de l'ester de départ (II) est 0,05 à 1,0M, le rapport molaire de l'ester (II) à l'alcool R⁵OH est d'au moins 1:0,5 ; la réaction étant conduite à une température de 0 à 60°C pendant un temps de 2 à 15 jours.

3. Un procédé selon la revendication 1 ou 2, dans lequel le groupe attirant les électrons présent dans R¹ est un groupe halogéno, nitro, cyano, -OR², -SR², -COR² (où R² peut être un groupe alkyle ou cycloalkyle en C₁-C₆), NR³R⁴ (où R³ et R⁴ peuvent être H, un groupe alkyle linéaire, ramifié ou cyclique en C₁-C₆, ou bien ils peuvent former un cycle en C₄-C₆ avec l'azote et facultativement d'autres hétéroatomes).

4. Un procédé selon l'une quelconque des revendications ci-dessus, dans lequel R¹ est -CH₂CF₃, -CH₂CH₂F, -CH₂CH₂Cl ou -CH₂CCl₃.

5. Un procédé selon l'une quelconque des revendications ci-dessus, dans lequel R¹ est -CH₂CF₃.

6. Un procédé selon l'une quelconque des revendications ci-dessus, dans lequel l'enzyme utilisée est la lipase de *Mucor meihei.*

7. Un procédé selon l'une quelconque des revendications ci-dessus, dans lequel l'alcool R⁵OH est un alcanol linéaire, ramifié ou cyclique en C₁-C₈ et l'ester IV (où R¹ a la même signification que dans la revendication la) est hydrolysé de façon chimiquement sélective par traitement avec un alcali aqueux à un pH constant de 7 à 12.

8. Un procédé selon la revendication 7, dans lequel l'alcool de formule générale R⁵OH est le *n*-butanol.

9. Un procédé selon la revendication 7 ou 8, dans lequel l'acide S et l'ester R finals résultants sont séparés par extraction avec un solvant organique non miscible à l'eau ou par centrifugation ou filtration de la suspension résultante.

10. Un procédé selon l'une quelconque des revendications 1 à 6 ci-dessus, dans lequel R⁵OH est un alcanol en C₁-C₄ substitué par des groupes ayant une fonctionnalité acide ou basique et l'ester n'ayant pas réagi IV (où R¹ a la même signification que dans la revendication la) ayant la configuration S est séparé par partage entre un solvant organique non miscible à l'eau et un milieu aqueux à un pH convenant pour dissoudre l'ester formé de formule III 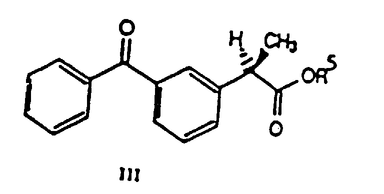 ou par centrifugation ou filtration de la suspension résultante, ce qui est suivi par une hydrolyse chimique ou enzymatique de l'ester IV.

11. Un procédé selon la revendication 10, dans lequel l'alcool R⁵OH est le 2-(2-pyridyl)éthanol.

12. Un procédé selon la revendication 10 ou 11, dans lequel les esters résultants sont séparés par partage entre un solvant organique non miscible à l'eau et une solution aqueuse à un pH acide de 0 à 4 ou un pH basique de 9 à 14.
